# EUROPEAN PATENT APPLICATION

(11) **EP 4 591 918 A1**
(43) Date of publication of application: **30.07.2025**
(21) Application number: 23868439.3
(22) Date of filing: 01.09.2023
(51) Int. Cl.: A61M 37/00

(54) **STAMP-TYPE DRUG DELIVERY DEVICE CAPABLE OF DELIVERING HIGH DOSE OF DRUG**

(30) Priority: 19.09.2022 KR 20220118087
(71) Applicant: Labnpeople Co.,Ltd., Yangju-si, Gyeonggi-do 11477 (KR)
(72) Inventor: CHO, Sung Youn, Uijeongbu-si Gyeonggi-do 11790 (KR); CHOI, Duk Su, Yangju-si Gyeonggi-do 11472 (KR)
(74) Representative: Maiwald GmbH
(86) International application number: PCT/KR2023/013110
(87) International publication number: WO 2024/063384

(57) **Abstract**

The present invention comprises: a needle part having a predetermined area and having a plurality of microneedles to be inserted into the skin of a user; an elastic restoration part elastically coming in contact with the needle part; a cartridge providing a space in which the needle part and the elastic restoration part can be accommodated, and having a plurality of slit-type openings; and a body which is coupled to the cartridge, and which moves downward by means of force transmitted from the user and presses the elastic restoration part.

## Description

### Technical Field

The present disclosure relates to a stamp-type drug delivery device capable of delivering a high dose of a drug. More particularly, the present disclosure relates to a stamp-type drug delivery device capable of easily and efficiently delivering a high dose of an effective component or a drug into the user's skin and increasing a thermal absorption rate.

### Background Art

In general, methods for delivering an effective component or drug into the body include taking a drug, injecting a drug, or applying the drug to the skin for absorption.

Of these methods, oral administration, or taking a drug by mouth, is limited to short-term use because this method can cause central nervous system effects, such as headache, drowsiness, dizziness, nervousness, etc., and serious gastrointestinal effects, such as nausea, dyspepsia, diarrhea, peptic ulcers, gastrointestinal bleeding, perforation, etc.

Furthermore, the injecting method delivers a drug directly into the body by injecting a needle through the skin, resulting in rapid absorption, but the method is cumbersome and injection by needle is only performed by experts (the method cannot be self-administered), and needles used in the method are several millimeters in diameter, resulting in low patient compliance due to the pain of the injection.

In order to eliminate the above-mentioned side effects and defects, drug delivery systems via dermal administration are being actively developed.

Usually, drugs that are absorbed through the skin are formulated in the form of liquids, creams, gels, etc., which are difficult to adjust a dose due to the nature of the formulation, and have problems such as stickiness or contamination of clothes. Furthermore, a problem with most drugs is that only a very small amount is absorbed and most of it is evaporated.

Therefore, a patch containing an effective component or drug is applied to the skin to deliver the drug subcutaneously.

The patch method has been increasingly used in recent years because absorption of the drug through the skin prevents side effects caused by taking the drug, such as gastrointestinal disorders and food interactions, and because there is the convenience of eliminating pain and discomfort during injection.

However, the patch method has a problem that the absorption rate of the drug through the skin is low. In other words, a major disadvantage of the patch is that a function of the skin as a permeation barrier must be reduced due to a low absorption rate of the drug through the skin. In general, it is known that the absorption rate of the drug through the patch is about 20%, and to date, chemical methods such as using skin absorption promoters and using prodrugs, and physical methods such as iontophoresis and electrophoresis are known to improve the absorption rate.

However, even using these known methods, there are still limitations in improving the drug absorption rate to a satisfactory level, so it is inevitable to use an excessive amount of drug in consideration of the absorption rate in order to expect pharmaceutical effects.

Specifically, a type of skin absorption promoter is determined by a design format of a formulation and physicochemical properties of components, and a certain level of concentration is required to be effective. When skin absorption promoters are added, it is not easy to prevent crystal formation over time, and adhesive properties such as adhesion and cohesion may be changed, unsuitable for use.

Furthermore, technologies that utilize electricity require patches to have cells, electrodes, circuits, and other components, which can lead to complex product configurations, large sizes, and high prices, as well as large amounts of waste after use.

Therefore, in order to increase the skin absorption rate of an effective component or drug, molten polymer micro-needles containing the effective component or drug have been developed, but it is still difficult to commercialize the micro-needles due to a low holding amount of drug, the low skin penetration efficiency of the micro-needles due to the limitations of the strength of the polymer, and the breakage of the micro-needles attached to the skin in an inclined plane rather than attached in a perpendicular direction when attached to the skin.

To solve the above problems, a method of forming and arranging a plurality of micro-needles on a thin bioresorbable metal plate with beneficial components to the human body and attaching them to the skin was proposed.

However, it is difficult to form and arrange the micro-needles within a limited planar area of a metal plate in order to maximize the effective component or drug delivery effect.

Also, the metal plate attached to the skin can be easily removed from the skin due to user's movement. Therefore, the adhesive patch is designed to prevent the metal plate from being removed from the skin, but it has a disadvantage of being psychologically rejected by a user because the adhesive patch and the metal plate have to be pressed against the skin for a certain period of time.

Furthermore, the micro-needles are made of a metal plate with a very thin thickness, so the needles can be easily broken or bent during injection into the skin.

Accordingly, the applicant has developed the present disclosure to address the above problems, and a related art reference include U.S. Application Publication No. 2007-0293815, "Microprojection array application with sculptured microprojections for high drug loading".

### Disclosure

### Technical Problem

To solve the above-mentioned problems, an objective of the present disclosure is to provide a stamp-type drug delivery device, which has a structure of preventing a plurality of micro-needles made of a metal plate from being broken or deformed when being injected into the skin and is configured to apply additional pressure after injection of the micro-needles to allow an effective component or drug to be delivered to the skin via the micro-needles.

Another objective of the present disclosure is to provide a stamp-type drug delivery device, which is configured to efficiently deliver an effective component or drug into the subcutaneous of a user without attachment of a metal plate with micro-needles on the skin of the user for a certain period of time, and specifically, to deliver a high dose of effective component or drug.

Yet another objective of the present disclosure is to provide a stamp-type drug delivery device, which is configured to arrange and form the density of micro-needles within a limited planar area of a metal plate to efficiently deliver an effective component or drug.

### Technical Solution

The present disclosure may include: a needle part having a predetermined area and having a plurality of micro-needles to be injected into skin of a user; an elastic restoration part elastically coming in contact with the needle part; a cartridge providing a space capable of accommodating the needle part and the elastic restoration part, and having a plurality of openings on a bottom part; and a body coupled to the cartridge and pressing the elastic restoration part by means of force transmitted from the user.

The needle part may include: a first needle sheet; first protrusions formed by cutting portions of an area of the first needle sheet, and protruding toward the openings of the cartridge; and a plurality of micro-needles provided at fore ends of the first protrusions.

When the plurality of micro-needles provided on the first protrusions is injected into the skin of the user, each first protrusion and each micro-needle formed on the first protrusion may be supportable by a first side surface constituting each opening of the cartridge.

The elastic restoration part may include: a first restoration means compressed by the body, in a state of being stacked at an upper portion of the first needle sheet; and a second restoration means disposed at a lower portion of the first needle sheet, and elastically supporting the lower portion of the first needle sheet.

The first restoration means may include: a first restoration sheet in which openings are formed to correspond to cutting holes formed in the first needle sheet; and a plurality of first elastic members formed by cutting a portion of an area of the first restoration sheet, and upwardly bent at a predetermined angle to come in elastic contact with a lower portion of the body.

The second restoration means may include: a second restoration sheet placed on a bottom surface of the cartridge, and having openings corresponding to the cutting holes formed in the first needle sheet; and a plurality of second elastic members formed by cutting a portion of an area of the second restoration sheet, and upwardly bent at a predetermined angle to come in elastic contact with a lower portion of the first needle sheet.

The second elastic members may have an elastic force or strength each less than an elastic force or strength of the first elastic members, or the second elastic members may be provided on the second restoration sheet fewer than the number of the first elastic members.

The body may include: a reservoir space in which an effective component or a drug is stored; a guide flow path connected to the reservoir space and through which the effective component or the drug stored in the reservoir space flows; and a discharge member guiding the effective component or the drug flowing through the guide flow path to the openings of the cartridge.

The stamp-type drug delivery device may include: a connection part of which a first portion is coupled to an upper end of the body and a second portion is coupled to the storage part in which an effective component or a drug is stored.

The needle part may include: a second needle sheet stacked on the first needle sheet; second protrusions formed by cutting a portion of an area of the second needle sheet and protruding toward the openings of the cartridge; and a plurality of micro-needles provided at fore ends of the second protrusions, wherein the second protrusions may be disposed to be spaced from the first protrusions at a predetermined distance.

When the plurality of micro-needles provided in the second protrusions is injected into the skin of the user, each second protrusion and each micro-needle formed in the second protrusion may be supportable by a second side surface partitioning the openings of the cartridge.

When the second needle sheet is stacked on an upper surface of the first needle sheet, the second protrusions may pass through cutting holes formed in the first sheet and is disposed to be spaced from the first protrusions at a predetermined distance, and when the first needle sheet is stacked on an upper surface of the second needle sheet, the first protrusions may pass through cutting holes formed in the second needle sheet and is disposed to be spaced from the second protrusions by a predetermined distance.

Each of the micro-needles of the needle part may include a slit in which an effective component or a drug is enabled to be contained or flow.

### Advantageous Effects

According to the embodiment of the present disclosure, the stamp-type drug delivery device capable of delivering a high dose of a drug is configured such that, when the plurality of micro-needles formed in the thin metal plate is injected into the skin, the first protrusion or the second protrusion supporting the micro-needles are supported by the inner surfaces partitioning the openings of the cartridge or by the discharge member of the body that is lowered to discharge the effective component or drug, thereby preventing breaking or deforming of the micro-needles.

Furthermore, according to the embodiment of the present disclosure, since the stamp-type drug delivery device capable of delivering a high dose of a drug has a structure that can prevent the plurality of micro-needles injected into the skin from being broken or deformed due to external force, the plurality of micro-needles can be made of thin metal plates, thereby increasing ease of manufacturing and decreasing cost of manufacturing thereof.

Furthermore, according to the embodiment of the present disclosure, since the stamp-type drug delivery device capable of delivering a high dose of a drug has the configuration of preferentially injecting the micro-needles disposed densely in a limited area into the skin, the configuration allows the effective component or drug to be efficiently delivered subcutaneously through the micro-needles and simultaneously to irritate the skin, which is a beneficial effect.

Furthermore, according to the embodiment of the present disclosure, the stamp-type drug delivery device capable of delivering a high dose of a drug is configured to preferentially inject the micro-needles into the user's skin by the first force of the user and to transfer a high dose of effective component or drug to a portion of the user's skin where the micro-needles are injected, by the second force of the user, so the thermal absorption rate of the effective component or drug can be increased.

Furthermore, according to the embodiment of the present disclosure, the stamp-type drug delivery device capable of delivering a high dose of drug includes the micro-needles to be injected into the skin, which is made of bioresorbable metal that provides beneficial effects on the skin, and therefore the device can provide effects of skin irritations and drug delivery, as well as subcutaneous swelling effect and a purpose as a drug delivery enhancer.

Furthermore, according to the embodiment of the present disclosure, the stamp-type drug delivery device capable of delivering a high dose of a drug may include a structure that can easily replace the micro-needles to be injected into the skin to correspond to the user, prevent infection, and be used semi-permanently.

### Description of Drawings

FIG. 1 is a perspective view showing a stamp-type drug delivery device according to an embodiment of the present disclosure.
FIG. 2 is an exploded-perspective view of the stamp-type drug delivery device according to the embodiment of the present disclosure.
FIG. 3 is a sectional view of the stamp-type drug delivery device according to the embodiment of the present disclosure.
FIG. 4 is a perspective view showing configuration of a needle part according to the embodiment of the present disclosure, the view being taken from the lower side.
FIG. 5 is a perspective view showing a state in which a first needle sheet and a second needle sheet of the needle part staked on each other, the view being taken from the lower side.
FIG. 6 is a perspective view showing configuration of an elastic restoration part according to the embodiment of the present disclosure.
FIG. 7 is a perspective view showing a first restoration means shown in FIG. 6 disposed at an upper surface of the second needle sheet, and a second restoration means disposed at a lower portion of the first needle sheet.
FIG. 8 is a side view showing the elastic restoration part shown in FIG. 7 and the needle part.
FIG. 9 is a perspective view showing a body according to the embodiment of the present disclosure.
FIG. 10 is a side view showing the body placed on first elastic members shown in FIG. 8.
FIG. 11 is a perspective view showing the needle part and the elastic restoration part accommodated in an inner space of a cartridge according to the embodiment of the present disclosure.
FIG. 12 is a perspective view showing the body placed on an upper portion of the elastic restoration part shown in FIG. 11.
FIG. 13 is a perspective view showing the cartridge shown in FIG. 12.
FIG. 14 is an enlarged view showing a micro-needle according to the embodiment of the present disclosure.
FIG. 15 is a perspective view showing the stamp-type drug delivery device according to another embodiment of the present disclosure.

### Mode for Invention

The above and other objects, features and advantages of embodiments of the present disclosure, and a method of achieving them will be more clearly understood with reference to the embodiments described below in detail in conjunction with the accompanying drawings.

However, the present disclosure is not limited to the following embodiments, and can be embodied in various forms different from each other, and embodiments of the present disclosure are presented to make complete disclosure of the present disclosure and help those who are ordinarily skilled in the art to which the present disclosure belongs understand the present disclosure. The present disclosure is only defined by the scope of the claims.

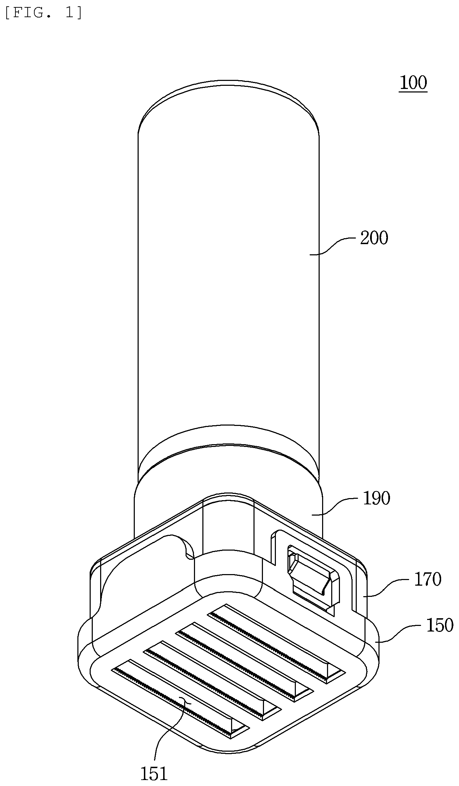

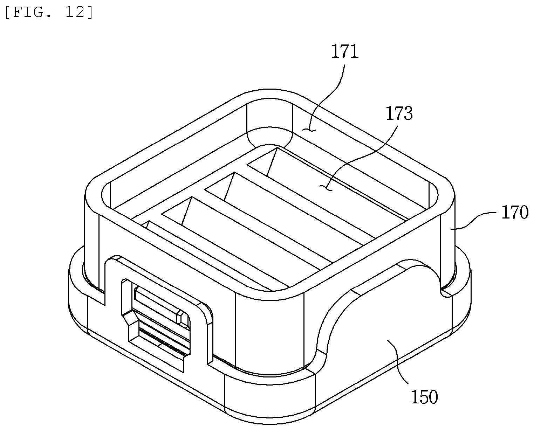

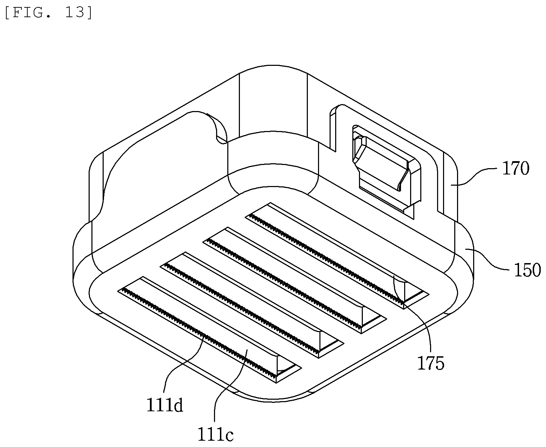

Hereinbelow, referring to FIGS. 1 to 15, according to the embodiment of the present disclosure, a stamp-type drug delivery device which is capable of delivering a high dose of a drug will be described in detail. In describing the present disclosure, specific descriptions for relevant known functions or configurations are omitted not to obscure the gist of the present disclosure.

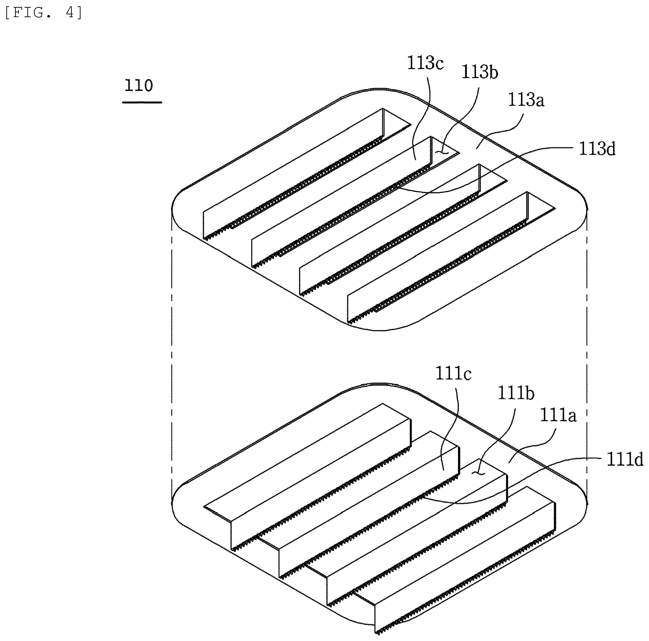

Referring to FIGS. 1 to 3, according to the embodiment of the present disclosure, a stamp-type drug delivery device 100 includes: a needle part 110 having a predetermined area and having a plurality of micro-needles 111d and 113d (referring to FIG. 4) injected into the skin of a user; an elastic restoration part 130 elastically coming in contact with the needle part 110; a cartridge 150 providing a space accommodatable for the needle part 110 and the elastic restoration part 130, and having a plurality of slit-type openings 151 on a bottom part; and a body 170 removably coupled to the cartridge 150, moved downward by means of force transmitted from the user and pressing the elastic restoration part 130, and providing a space through which an effective component or a drug to be delivered to the skin of the user passes.

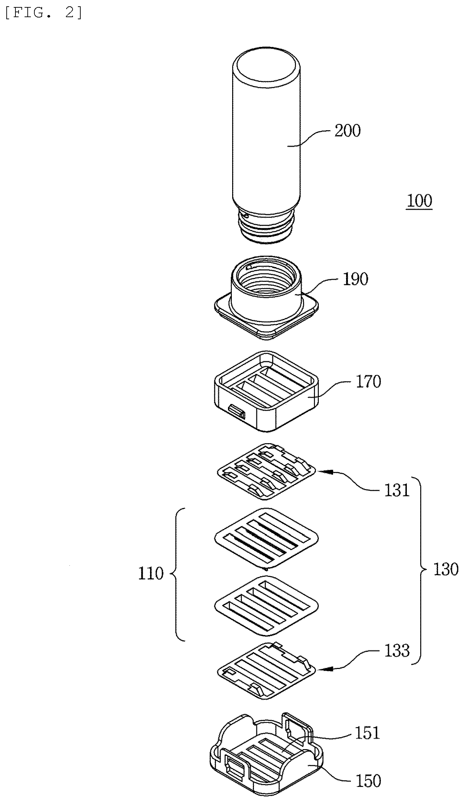

First, as shown in FIGS. 2, 4, and 5, the needle part 110 may include a plurality of needle sheets 111a and 113a.

For reference, in the embodiment of the present disclosure, the needle part 110 is shown in the view as consisting of a first needle sheet 111a and a second needle sheet 113.

The first needle sheet 111a is a component mounted to the inner space of the cartridge 150, and as shown in FIG. 4, the first needle sheet 111a includes a first protrusion 111c and the micro-needles 111d.

The first protrusion 111c may be a component formed by cutting out a portion of an area of the first needle sheet 111a. In other words, the first protrusion 111c may be a component formed by cutting a portion of an area of the first needle sheet 111a and then folding it in one direction.

Therefore, the first needle sheet 111a includes cutting holes 111b to form the first protrusion 111c. The cutting holes 111b may have locations and sizes corresponding to discharge holes 151 formed in the cartridge 150.

Referring to the drawing, the embodiment of the present disclosure is shown with the first needle sheet 111a having four first protrusions 111c and four cutting holes 111b. However, without being limited thereto, obviously, the number of first protrusions 111c and the number of cutting holes 111b are variously adjustable within an area formed from the first needle sheet 111a.

Meanwhile, the first protrusions 111c may protrude toward the openings 151 formed in the cartridge 150 when the first needle sheet 111a is disposed inside the cartridge 150. At this point, the first protrusions 111c may be disposed in a vertical direction or an inclined direction and protrude toward the openings 151. For reference, in the embodiment of the present disclosure, it is shown that the first protrusions 111c are positioned vertically.

A plurality of micro-needles 111d may be formed on a fore end of each first protrusion 111c. The micro-needles 111d may be a component that is directly injected into the skin of the user, and a plurality of micro-needles 111d may be formed at predetermined intervals in a longitudinal direction of the first protrusions 111c.

When the plurality of micro-needles 111d formed in each first protrusion 111c is injected into the skin of the user, the micro-needles 111d formed in the first protrusions 111c and the first protrusions 111c may be supported by first side surfaces partitioning the openings 151 of the cartridge 150.

In other words, in a process of injecting the micro-needles 111d formed in the first protrusions 111c into the skin, when each first protrusion 111c or the plurality of micro-needles 111d is broken or bent, it may be brought into contact with and supported by a first side surface partitioning each opening 151 of the cartridge 150.

Therefore, the plurality of micro-needles 111d of the first needle sheet 111a to be injected into the skin, because each first protrusion 111c can be supported by the first side surface of each opening 151, may be easily injected into the skin without being broken or deformed due to external force.

Also, the second needle sheet 113a is a component mounted to the inner space of the cartridge 150, and as shown in FIG. 4, the second needle sheet 113a includes second protrusions 113c and micro-needles 113d.

The second needle sheet 113a may be a component that is brought into contact with and stacked on an upper surface of a lower surface of the first needle sheet 111a. In the embodiment of the present disclosure, it is described that the second needle sheet 113a is stacked on the upper surface of the first needle sheet 111a, and the view illustrates that the second needle sheet 113a is disposed on the upper surface of the first needle sheet 111a.

The second protrusion 113c may be a component formed by cutting out a portion of an area of the second needle sheet 113a. In other words, the second protrusion 113c may be a component formed by cutting a portion of an area of the first needle sheet 111a and then folding it in one direction.

Therefore, the second needle sheet 113a includes cutting holes 113b to form the second protrusion 113c. The cutting holes 113b formed in the second needle sheet 113a may have locations and sizes corresponding to the locations and sizes of the cutting holes 111b formed in the first needle sheet 111a.

Referring to the drawing, the embodiment of the present disclosure is shown with the second needle sheet 113a having four second protrusions 113c and four cutting holes 113b. However, without being limited thereto, the second needle sheet 113a obviously has the second protrusions 113c and the cutting holes 113b to correspond to the number of the number of first protrusions 111c and the number of cutting holes 111c formed in the first needle sheet 111a.

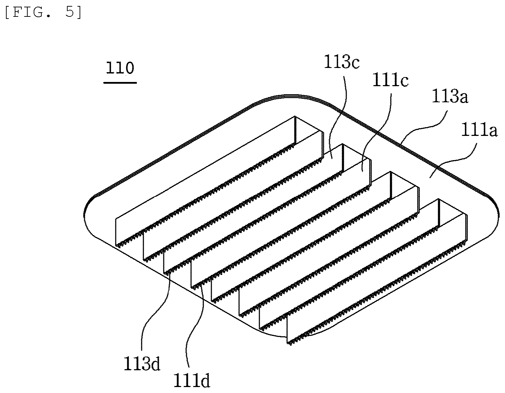

As shown in FIGS. 4 and 5, when the second needle sheet 113a is staked on the upper surface of the first needle sheet 111a, the second protrusions 113c pass through the cutting holes 111b formed in the first needle sheet 111a and are disposed to be spaced from the first protrusions 111c at predetermined intervals.

On the other hand, although not shown in the drawings, when the first needle sheet 111a is stacked on an upper surface of the second needle sheet 113a, the first protrusions 111c of the first needle sheet 111a may pass through the cutting holes 113b formed in the second needle sheet 113a and are disposed to be spaced from the second protrusions 113c at predetermined intervals.

Meanwhile, the second protrusions 113c may protrude toward the openings 151 formed in the cartridge 150. At this point, the second protrusions 113c may be disposed in a vertical direction or an inclined direction and protrude toward the openings 151. For reference, in the embodiment of the present disclosure, it is shown that the second protrusions 113c are also positioned vertically.

The plurality of micro-needles 113d of the second needle sheet 113a may be formed on a fore end of each second protrusion 113c. The micro-needles 111d may be a component that is directly injected into the skin of the user, and a plurality of micro-needles 111d may be formed at predetermined intervals in a longitudinal direction of the second protrusions 113c.

When the plurality of micro-needles 113d formed in each second protrusion 113c is injected into the skin of the user, the micro-needles 113d formed in the second protrusions 113c and the second protrusions 113c may be supported by second side surfaces partitioning the openings 151 formed in the cartridge 150.

In other words, in a process of injecting the micro-needles 113d formed in the second protrusions 113c into the skin, when each second protrusion 113c or the plurality of micro-needles 113d is broken or bent, it may be brought into contact with and supported by a second side surface partitioning each opening 151 of the cartridge 150.

Therefore, the plurality of micro-needles 113d of the second needle sheet 113a to be injected into the skin, because each second protrusion 113c can be supported by the second side surface of each opening 151, may be easily injected into the skin without being broken or deformed due to external force.

The second needle sheet 113a configured as described above serves to increase the density of the micro-needles 111d and 113d within a limited planar space.

In other words, within a limited planar space formed by the cartridge 150, the density and the number of micro-needles 111d and 113d increase to expand a needle injection area where the needles are brought into contact with the skin of the user, thereby increasing the subcutaneous delivery effect of the effective component or the drug.

In the embodiment of the present disclosure, it is described and shown that the two first needle sheet 111a and second needle sheet 113a are stacked on each other and are provided inside the cartridge 150, but the embodiment of the present disclosure is not limited thereto. For example, one needle sheet may be used alone, and to increase the density of the needles, two or more needle sheets may be stacked on each other and used.

Furthermore, the first needle sheet 111a and the second needle sheet 113a may be made of metal including at least one of magnesium, calcium, zinc, and iron utilized as bioresorbable metal. Likewise, the protrusions 111c and 113c and the micro-needles 111d and 113d provided on the first needle sheet 111a and the second needle sheet 113a may be made of metal including at least one of magnesium, calcium, zinc, and iron utilized as bioresorbable metal.

For reference, for application of bioresorbable metal as orthopedic implants, magnesium-based alloys have been produced and commercialized at home and abroad, and bioresorbable metals applied to orthopedic implants focus on reducing a rate of degradation in the body as much as possible or improving corrosion resistance for safe fracture fixation.

However, according to the embodiment of the present disclosure, bioresorbable metal forming the first needle sheet 111a, the second needle sheet 113a, the protrusions 111c and 113c, and the micro-needles 111d and 113d may use a mechanism of accelerating a rate of degradation in the body, allowing mineral to be supplied subcutaneously with release of a drug, unlike bioresorbable metals applied in orthopedic applications.

For example, magnesium, calcium, and zinc that are used as bioresorbable metal may have a mechanism to react with water in the body to release hydrogen gas and break down. Therefore, when the micro-needles 111d and 113d provided in the first needle sheet 111a and the second needle sheet 113a are inserted into the skin, the needles release ions and degradation products in the skin, and one of its byproducts, hydrogen gas, can provide a swelling effect in the skin, which can also induce anti-wrinkle effects.

Furthermore, ZnO, MgCl, which are by-products of magnesium and zinc, i.e., components of bioresorbable metals, inserted into the human body, can also serve as a drug delivery enhancer, which enhances the absorption of the effective component or the drug into the skin. Therefore, the first needle sheet 111a and the second needle sheet 113a made of bioresorbable metal may have a function of efficiently delivering the effective component or drug held therein or an effective component or drug transferred from the outside part, subcutaneously.

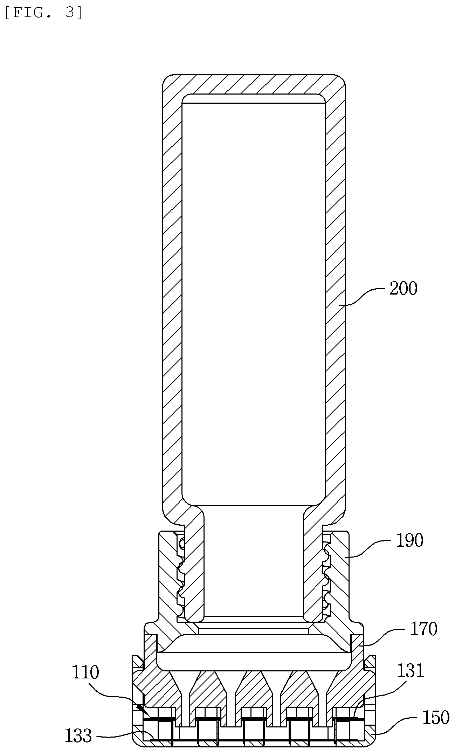

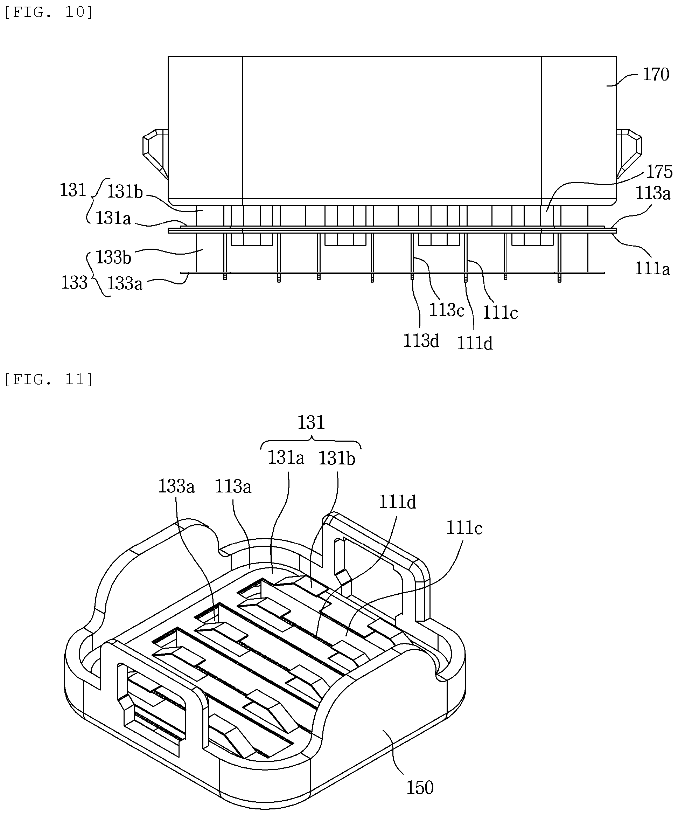

As shown in FIGS 3 and 11, the elastic restoration part 130 may be disposed with the needle part 110 in the internal space formed by the cartridge 150, and may be configured to be compressed by receiving a pressing force transmitted from the body 170.

In addition, the elastic restoration part 130 may be configured to be compressed by a force transmitted from the user or to be restored to the original state. For example, when the user applies a force, the elastic restoration part 130 may transmit the force to the needle part 110. Thereafter, the micro-needles 111d and 113d provided in the needle part 110 may be exposed outwards through the openings 151 of the cartridge 150. On the other hand, when the force from the user is removed, the elastic restoration part 130 provides a restoration force that allows the micro-needles 111d and 113d that are injected into the user's skin through the openings 151 of the cartridge 150 to be restored to the original state.

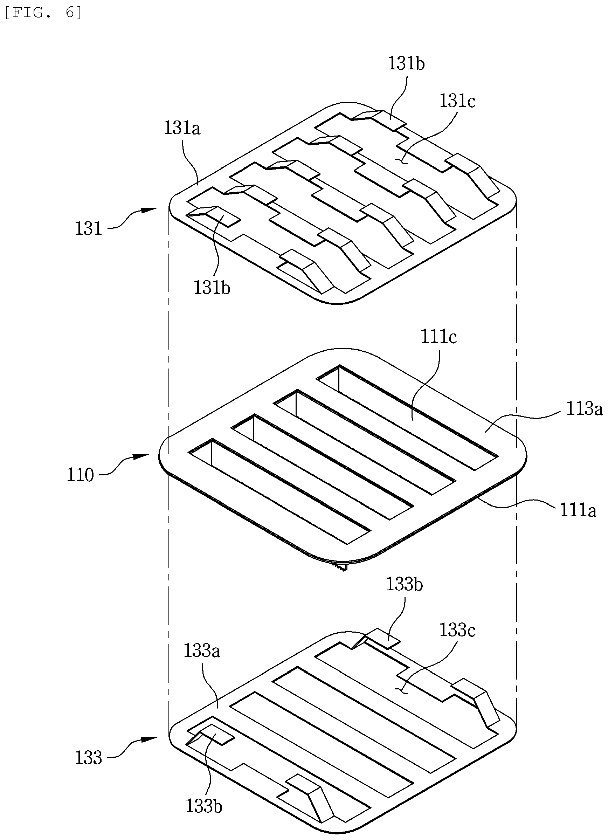

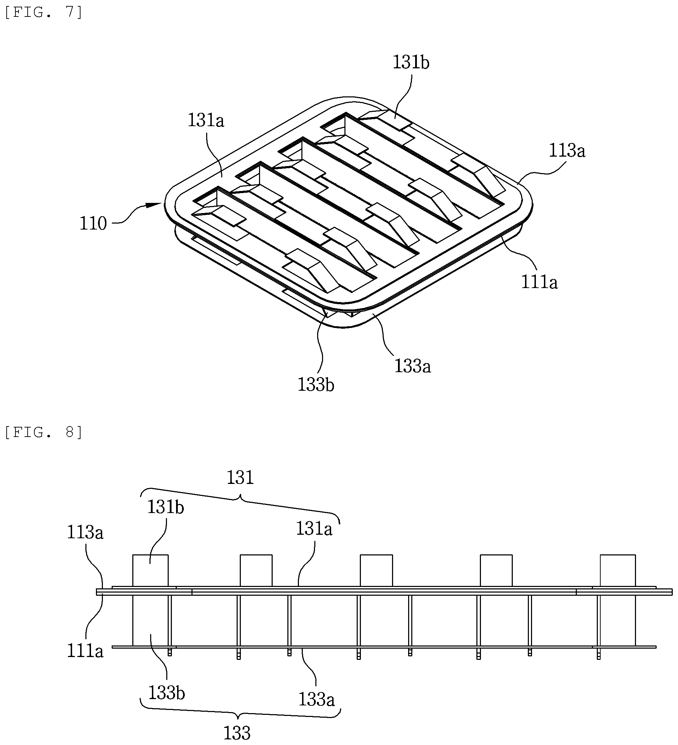

As shown in FIGS. 6 and 8, the elastic restoration part 130 having the above-described functions may include a first restoration means 131 and a second restoration means 133.

The first restoration means 131 is a component disposed at an upper portion of the needle part 110. In the embodiment of the present disclosure, the first restoration means 131 may be stacked on an upper portion of the second needle sheet 113a and compressed from a lower portion of the body 170.

The second restoration means 133 is a component disposed at a lower portion of the needle part 110. In the embodiment of the present disclosure, the second restoration means 133 may be placed on a bottom surface of the cartridge 150 to elastically support a lower portion of the first needle sheet 111a.

First, the first restoration means 131 may include a first restoration sheet 131a in which the openings 131c are formed with locations and sizes corresponding to the cutting holes 111b formed in the first needle sheet 111a; and a plurality of first elastic members 131b formed by cutting a portion of an area of the first restoration sheet 131a, bent upwards at a predetermined angle, and elastically coming in contact with the lower portion of the body 170.

The second restoration means 133 may include a second restoration sheet 133a placed on a bottom surface of the cartridge 150, and having openings 133c that are formed to correspond to the cutting holes 111b formed in the first needle sheet 111a; and a plurality of second elastic members 133b formed by cutting a portion of an area of the second restoration sheet 133a, bent upwards at a predetermined angle, and elastically coming in contact with the lower portion of the first needle sheet 111a.

The first restoration sheet 131a and the second restoration sheet 133a may have a planar area smaller than a planar area of the first needle sheet 111a or the second needle sheet 113a.

Furthermore, the first restoration sheet 131a and the second restoration sheet 133a may be made of a metal or plastic material that has an elastic restoration force.

When the body 170 is moved vertically downwards, the first elastic members 131b may be hinged to be at the same height as the height of the first restoration sheet 131a when being pressed from the lower portion of the body 170. On the other hand, when the force transmitted to the body 170 is removed, the first elastic members 131b may protrude upwards on the first restoration sheet 131a to be restored to the original state.

The first elastic members 131b may protrude upwards on the first restoration sheet 131a to be inclined and protrude again in a horizontal direction. This shape facilitates the first elastic members 131b to receive pressure from the lower portion of the body 170. Furthermore, when the force transmitted to the body 170 is removed, this shape facilitates the first elastic members 131b to be rotated on the first restoration sheet 131a and be restored to the original state.

When the body 170 is lowered vertically, the second elastic members 133b may receive pressure from a lower portion of the lowest needle sheet 111a, 113a, among the plurality of needle sheets 111a and 113a constituting the needle part 110, and then be hinged to be at the same height as the height of the second restoration sheet 133a. For reference, in the embodiment of the present disclosure, since the second elastic members 133b come into contact with the lower portion of the first needle sheet 111a, so the view shows that when the body 170 is vertically lowered, the second elastic members 133b receives pressure from the first needle sheet 111a. On the other hand, when the force transmitted to the body 170 is removed, the second elastic members 133b may protrude upwards on the second restoration sheet 133a to be restored to the original state.

The second elastic members 133b may protrude upwards on the second restoration sheet 133a to be inclined and protrude again in a horizontal direction. This shape facilitates the second elastic members 133b to receive pressure from the lower portion of the first needle sheet 111a or the second needle sheet 113a. Furthermore, when the force transmitted to the body 170 is removed, this shape allows the second elastic members 133b to be rotated on the second restoration sheet 133a and to be restored to the original state.

Meanwhile, the second elastic members 133b may have the elastic force or strength less than or equal to the elastic force or strength of the first elastic members 131b. Otherwise, the second elastic members 133b may be provided fewer than the number of first elastic members 131b, on the second restoration sheet 133a.

Accordingly, when a first force applied from the user is transmitted to the body 170 to allow the body 170 to be lowered, the second elastic members 133b provided on the second restoration sheet 133a may be compressed in preference to the first elastic members 131b provided on the first restoration sheet 131a.

Accordingly, the micro-needles 111d and 113d of the needle part 110 may be exposed by the first force applied from the user through the openings 151 of the cartridge 150 and injected into the skin of the user.

Then, when a second force stronger than the first force is generated, the body 170 is further lowered, thereby compressing the first elastic members 131b of the first restoration sheet 131a. Therefore, as discharge members 175 of the body 170, which will be described below, are disposed to be adjacent to the skin of the user, a high dose of effective component or drug may be quickly discharged into the skin of the user.

As described above, the first restoration means 131 and the second restoration means 133 may be compressed or restored with a time interval as the elasticity, strength, forming numbers of the first elastic members 131b and the second elastic members 133b are preset differently from each other. Therefore, the user appropriately adjusts a pressing force to realize each function of the drug delivery device according to the dual operation. For example, the first operation of applying the first force may allow the micro-needles 111d and 113d to be preferentially injected into the skin, and the second operation of applying the second force may allow a high dose of effective component or drug to be transferred to the skin.

As shown in FIGS. 11 to 13, the cartridge 150 may form a predetermined space where the needle part 110 and the elastic restoration part 130 may be accommodated.

Furthermore, the plurality of openings 151 may be formed in the bottom part of the cartridge 150.

The openings 151 formed in the bottom part of the cartridge 150 may have various forms and sizes. In the embodiment of the present disclosure, the view shows four openings 151 that are formed to be spaced from each other at predetermined intervals.

Furthermore, as the forms, sizes, and number of openings 151 formed in the cartridge 150 may be as the standard for the forms, sizes, and number of the cutting holes 111b and 113b constituting the needle part 110 and the forms, sizes, and number of the openings 131c and 133c constituting the elastic restoration part 130.

Furthermore, longitudinal portions of the first protrusions 111c, and longitudinal portions of the second protrusions 113c, and the micro-needles 111d and 113d may be exposed outwards through the openings 151.

As shown in FIG. 11, the body 170 may be attachably and detachably coupled to the cartridge 150.

It is obvious that the method for attachably and detachably coupling the body 170 and the cartridge 150 to each other may be a variety of known methods. For reference, a ring part is formed in the cartridge 150, and a protrusion injected into and caught by the ring part is formed at the body 170, so the body 170 and the cartridge 150 may be coupled to each other to be attachable and detachable from each other.

For reference, a reason of attachably and detachably coupling the body 170 and the cartridge 150 to each other is to enable the user to easily replace the needle part 110 and the elastic restoration part 130 provided in the internal space of the cartridge 150.

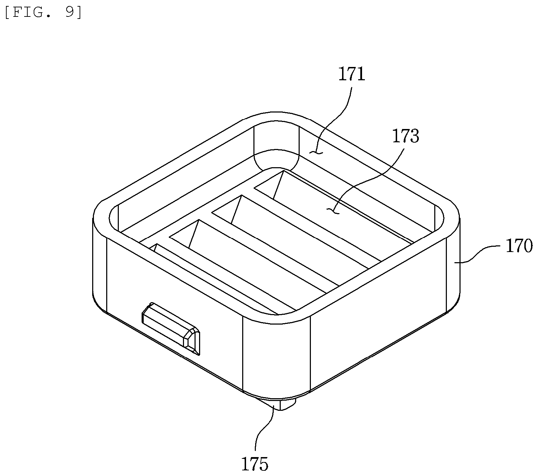

As shown in FIGS. 9 and 10, the body 170 may include a reservoir space 171 in which the effective component or drug is stored; guide flow paths 173 connected to the reservoir space 171 and allowing the effective component or drug stored in the reservoir space 171 to flow; and the discharge members 175 guiding the effective component or drug flowing along the guide flow paths 173 to the openings 151 of the cartridge 150.

The reservoir space 171 may be a space where the effective component or drug discharged from a storage part 200 (referring to FIGS. 2 and 3) is held for a predetermined time.

For reference, as shown in FIGS. 2 and 3, the body 170 and the storage part 200 may be connected to each other by a medium of a connection part 190. In other words, the connection part 190 may have a hollow form, and a first portion thereof may be attachably and detachably coupled to an open upper portion of the body 170. Furthermore, a second portion of the connection part 190 may be attachably and detachably coupled to the storage part 200.

The method for attachably and detachably coupling the connection part 190 to the body 170 or the storage part 200 may be various known coupling methods. For example, various methods such as a forced-fitting method, a screwing method, etc., may be used.

Furthermore, the storage part 200 may provide a space for storing the effective component or drug and have a predetermined strength. The storage part 200 may be made in various types such as a container type, a capsule type, etc.

Therefore, the effective component or drug discharged from the storage part 200 may flow through the connection part 190 and stay temporarily in the reservoir space 171 of the body 170.

The effective component or drug staying in the reservoir space 171 may be introduced into the guide flow paths 173.

The guide flow paths 173 may be provided in the body 170 with locations and the number corresponding to the locations and the number of the openings 151 formed in the cartridge 150.

The guide flow paths 173 may be formed in the body 170 with a shape that gradually narrows a space therein to increase a flow speed of the effective component or drug stored in the reservoir space 171. For example, the guide flow paths 173 may be partitioned by a pair of inclined surfaces.

The discharge members 175 may be connected to the guide flow paths 173 to communicate with each other. As shown in FIG. 10, each of the discharge members 175 may have a shape protruding from a lower surface of the body 170.

The discharge members 175 may be successively injected into the openings 131c formed in the first restoration sheet 131a, the cutting holes 111b and 113b formed in the needle part 110, and the openings 133c formed in the second restoration sheet 133a.

In other words, when the body 170 is lowered, the discharge members 175 may pass successively through the openings 131c of the first restoration sheet 131a, the cutting holes 111b and 113b of the needle part 110, and the openings 133c of the second restoration sheet 133a and then be disposed at locations corresponding to the openings 151 formed in the cartridge 150. In this state, the effective component or drug flowing through the guide flow paths 173 may be quickly delivered to the skin.

At this point, the discharge members 175 pass through spaces formed between the first protrusions 111c formed in the first needle sheet 111a and the second protrusions 113c formed in the second needle sheet 113a, whereby sweeping off the drug applied on the first protrusions 111c and the second protrusions 113c and quickly delivering the drug to the skin of the user.

In addition, since the discharge members 175 are disposed between the first protrusions 111c and the second protrusions 113c, the discharge members 175 may support the first protrusions 111c and the second protrusions 113c when the plurality of micro-needles 111d formed in the first needle sheet 111a and the plurality of micro-needles 113d formed in the second needle sheet 113a are injected into the skin.

The discharge members 175 are operated in conjunction with first surfaces partitioning the openings 151 of the cartridge 150 to prevent the first protrusions 111c and the micro-needles 111d of the first needle sheet 111a from being broken or deformed in a second direction and, in addition, operated with conjunction with second surfaces partitioning the openings 151 of the cartridge 150 to prevent the second protrusions 113c and the micro-needles 113d of the second needle sheet 113a from being broken or deformed in a first direction.

In other words, the discharge members 175 may prevent the space in advance that allows the first protrusions 111c and the second protrusions 113c to be broken or deformed.

Therefore, the discharge members 175 may serve to prevent unintentionally breaking or deforming of the micro-needles 111d and 113d having a micro unit during the micro-needles are injected into the skin.

According to the embodiment of the present disclosure having the above-described configuration, the stamp-type drug delivery device is configured such that, when the plurality of micro-needles formed in the thin metal plate is injected into the skin, the first protrusion or the second protrusion supporting the micro-needles are supported by the inner surfaces partitioning the openings of the cartridge or by the discharge member of the body that is lowered to discharge the effective component or drug, thereby preventing breaking or deforming of the micro-needles.

Furthermore, since there is provided the configuration of preferentially injecting the micro-needles that are densely disposed in a limited area, into the skin, the configuration allows the effective component or drug to be efficiently delivered subcutaneously through the micro-needles and simultaneously to irritate the skin, which is a beneficial effect.

Although the specific embodiment of the present disclosure has been described herein, it is obvious that various modifications, equivalents, additions and substitutions are possible without departing from the scope of the present disclosure.

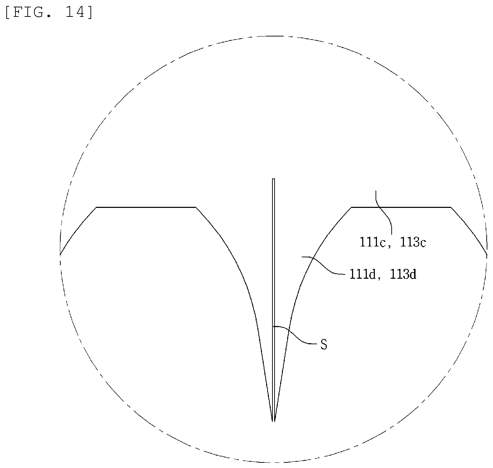

For example, as shown in FIG. 14, to increase the subcutaneous delivery effect of the effective component or drug, each micro-needle 111d, 113d may include a slit S in which the effective component or drug may be held or flow.

The slit S may be formed in a direction that is parallel to an injecting direction of each micro-needle 111d, 113d and serve to allow the effective component or drug to be quickly and efficiently transferred into the skin.

Of course, the length, width, and formation location of the slit S may be variously preset to be the form and size of each micro-needle 111d, 113d.

Furthermore, according to the embodiment of the present disclosure, the stamp-type drug delivery device 100 has been described to deliver the effective component or drug stored in the storage part 200 to the skin sequentially through the connection part 190, the body 170, the needle part 110, and the cartridge 150, but is not limited thereto.

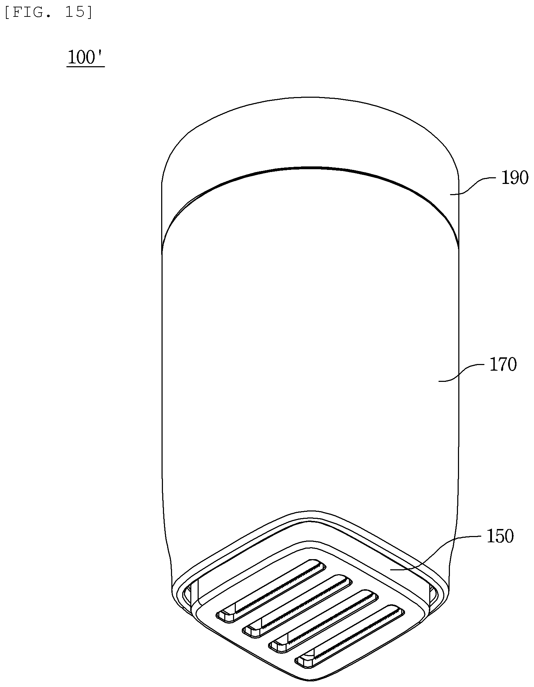

For example, as shown in FIG. 15, the storage part 200 of storing the effective component or drug may be omitted. Instead of the storage part, the above-described body 170 may be changed into a form and a volume that can store the effective component or drug, and the above-described connection part 190 may serve as a cap that covers the open upper portion of the body 170.

Furthermore, according to the embodiment of the present disclosure, the needle part 110 of the stamp-type drug delivery device 100 is made of metal containing beneficial components to the human body, and the micro-needles 111d and 113d have a beneficial effect on the user by themselves.

Therefore, the user can only use the stamp-type drug delivery device 100 according to the present disclosure for the purpose of injecting the micro-needles 111d and 113d into the skin, and in this case, the storage part 200 that is a component for storing the effective component or drug may be omitted. At this point, the body 170 serves to transmit the user's force to the needle part 110 and simultaneously to prevent breaking or deforming of the first protrusions 111c, the second protrusions 113c, and the micro-needles 111d and 113d when the micro-needles 111d and 113d are injected into the skin.

Therefore, the scope of the present disclosure should not be limited to the above-described embodiment and should be defined by the scope of the accompanying claims as well as by equivalents to the scope of the claims.

## Claims

1. A stamp-type drug delivery device capable of delivering a high dose of a drug, the stamp-type drug delivery device comprising:
a needle part having a predetermined area and having a plurality of micro-needles to be injected into skin of a user;
an elastic restoration part elastically coming in contact with the needle part;
a cartridge providing a space capable of accommodating the needle part and the elastic restoration part, and having a plurality of openings on a bottom part; and
a body coupled to the cartridge and pressing the elastic restoration part by means of force transmitted from the user.

2. The stamp-type drug delivery device of claim 1, wherein the needle part comprises:
a first needle sheet;
first protrusions formed by cutting portions of an area of the first needle sheet, and protruding toward the openings of the cartridge; and
a plurality of micro-needles provided at fore ends of the first protrusions.

3. The stamp-type drug delivery device of claim 2, wherein when the plurality of micro-needles provided on the first protrusions is injected into the skin of the user, each first protrusion and each micro-needle formed on the first protrusion are supportable by a first side surface constituting each opening of the cartridge.

4. The stamp-type drug delivery device of claim 2, wherein the elastic restoration part comprises:
a first restoration means compressed by the body, in a state of being stacked at an upper portion of the first needle sheet; and
a second restoration means disposed at a lower portion of the first needle sheet, and elastically supporting the lower portion of the first needle sheet.

5. The stamp-type drug delivery device of claim 4, wherein the first restoration means comprises:
a first restoration sheet in which openings are formed to correspond to cutting holes formed in the first needle sheet; and
a plurality of first elastic members formed by cutting a portion of an area of the first restoration sheet, and upwardly bent at a predetermined angle to come in elastic contact with a lower portion of the body.

6. The stamp-type drug delivery device of claim 5, wherein the second restoration means comprises:
a second restoration sheet placed on a bottom surface of the cartridge, and having openings corresponding to the cutting holes formed in the first needle sheet; and
a plurality of second elastic members formed by cutting a portion of an area of the second restoration sheet, and upwardly bent at a predetermined angle to come in elastic contact with a lower portion of the first needle sheet.

7. The stamp-type drug delivery device of claim 6, wherein the second elastic members have an elastic force or strength each less than an elastic force or strength of the first elastic members, or the second elastic members are provided on the second restoration sheet fewer than the number of the first elastic members.

8. The stamp-type drug delivery device of claim 2, wherein the body comprises:
a reservoir space in which an effective component or a drug is stored;
a guide flow path connected to the reservoir space and through which the effective component or the drug stored in the reservoir space flows; and
a discharge member guiding the effective component or the drug flowing through the guide flow path to the openings of the cartridge.

9. The stamp-type drug delivery device of claim 1, further comprising:
a connection part of which a first portion is coupled to an upper end of the body and a second portion is coupled to the storage part in which an effective component or a drug is stored.

10. The stamp-type drug delivery device of claim **2,** wherein the needle part comprises:
a second needle sheet stacked on the first needle sheet;
second protrusions formed by cutting portions of an area of the second needle sheet and protruding toward the openings of the cartridge; and
a plurality of micro-needles provided at fore ends of the second protrusions,
wherein the second protrusions are disposed to be spaced from the first protrusions at a predetermined distance.

11. The stamp-type drug delivery device of claim 10, wherein when the plurality of micro-needles provided in the second protrusions is injected into the skin of the user, each second protrusion and each micro-needle formed in the second protrusion are supportable by a second side surface partitioning the openings of the cartridge.

12. The stamp-type drug delivery device of claim 10, wherein when the second needle sheet is stacked on an upper surface of the first needle sheet, the second protrusions pass through cutting holes formed in the first sheet and is disposed to be spaced from the first protrusions at a predetermined distance, and
when the first needle sheet is stacked on an upper surface of the second needle sheet, the first protrusions pass through cutting holes formed in the second needle sheet and are disposed to be spaced from the second protrusions by a predetermined distance.

13. The stamp-type drug delivery device of claim 10, wherein each of the micro-needles of the needle part comprises a slit in which an effective component or a drug is enabled to be contained or flow.
